# EUROPEAN PATENT APPLICATION

(11) **EP 3 747 896 A1**
(43) Date of publication of application: **09.12.2020**
(21) Application number: 19747024.8
(22) Date of filing: 31.01.2019
(51) Int. Cl.: C07K 14/62, C07K 1/107, A61P 3/10, A61K 38/28

(54) **PHARMACEUTICAL COMPOSITION COMPRISING ACYLATED DERIVATIVE OF HUMAN INSULIN ANALOG AND PREPARATION METHOD THEREOF**

(30) Priority: 01.02.2018 CN 201810099660
(71) Applicant: Jiangsu Hengrui Medicine Co., Ltd., Lianyungang, Jiangsu 222047 (CN)
(72) Inventor: YANG, Xiaorong, Lianyungang, Jiangsu 222047 (CN)
(74) Representative: dompatent von Kreisler Selting Werner - Partnerschaft von Patent- und Rechtsanwälten mbB
(86) International application number: PCT/CN2019/074146
(87) International publication number: WO 2019/149245

(57) **Abstract**

A composition comprising a human insulin analog. In particular, provided is a composition comprising an acylated derivative of a human insulin analog. The acylated derivative composition is more stable than an existing insulin analog and an acylated derivative composition thereof.

SEQUENCE LISTING

<110>  JIANGSU HENGRUI MEDICINE CO., LTD.

<120>  Pharmaceutical composition comprising acylated derivative of
       human insulin analog and preparation method thereof

<130>  202014EP

<140>  PCT/CN2019/074146
<141>  2019-01-31

<150>  CN201810099660.8
<151>  2018-02-01

<160>  2

<170>  PatentIn version 3.5

<210>  1
<211>  21
<212>  PRT
<213>  Homo sapiens

<400>  1

Gly Ile Val Glu Gln Cys Cys Thr Ser Ile Cys Ser Leu Tyr Gln Leu
1               5                   10                  15


Glu Asn Tyr Cys Asn
            20



<210>  2
<211>  29
<212>  PRT
<213>  Artificial Sequence

<220>
<223>  Mutant human insulin with deletion at position 29 of the B chain

<400>  2

Phe Val Asn Gln His Leu Cys Gly Ser His Leu Val Glu Ala Leu Tyr
1               5                   10                  15


Leu Val Cys Gly Glu Arg Gly Phe Phe Tyr Thr Pro Lys
            20                  25

## Description

The present invention claims the priority of the Chinese patent application CN201810099660.8, filed on February 1, 2018, the contents of which are incorporated herein by its entirety.

### Field of invention

The present invention relates to a pharmaceutical composition comprising an acylated derivative of human insulin analog and a preparation method thereof.

### Prior arts

As a first line drug for treating diabetes, human insulin has a short duration of action, which makes frequent injections necessary and cause extremely inconvenience to patients. Therefore, people are committed to obtaining some insulin analogs and derivatives thereof with a longer half-life and long term action on human body. Among them, the modification of human insulin or analogs thereof with acylated groups is an effective method to increase the half-life of insulin. The applicant's patent application PCT/CN2017/095377 provides a human insulin analog with position B29 substituted with a long-chain fatty acid and amino acid at position B30 deleted, and discloses the structure and biological activity of the human insulin analog. WO9507931 discloses an insulin (i.e. Des(30) human insulin) analog with B29 position linked to a tetradecyl side chain and amino acid at position B30 deleted, and a preparation thereof. WO2005012347 discloses a human insulin analog with B29 position substituted with a glutamic acid and a long-chain fatty acid and amino acid at position B30 deleted. WO2007074133 discloses a preparation of the above insulin analogs, and LysB29 (Nε-hexadecanodiacyl-γ-glutamyl de(B30) human insulin (insulin degludec) preparation disclosed in the patent contains sodium chloride. However, the information published by FDA (https://www.accessdata.fda.gov/drugsatfda_docs/nda/2015/2033130rig1s000_2033140rig 1s000PharmR.pdf) shows that sodium chloride will lead to a decrease in system exposure of insulin degludec. In order to solve the above problem, sodium chloride has been removed from the product preparation of insulin degludec in the market.

The decrease in system exposure of insulin leads to a reduction in the amount of insulin that can actually work, thereby reducing its hypoglycemic effect. The present invention surprisingly finds that for the acylated derivatives of insulin of the present invention, the liquid preparation containing sodium chloride can not only reduce the production of high molecular weight polymers, but also have no influence on the hypoglycemic effect of insulin.

### Content of the present invention

The present invention provides a pharmaceutical composition comprising an acylated derivative of human insulin analog, wherein the acylated derivative of human insulin analog has a structure represented by the following general formula I:

S-W-X-Y-Z (I)

wherein S is a human insulin with threonine deletion at position 30 of the B chain (deletion of the B chain is shown in SEQ ID NO.2), i.e. Des(B30) human insulin; -W-X-Y-Z is an acylation modification group of the insulin analog;
wherein W is a diacyl structure with -OC(CH₂)ₙCO-, where n is an integer between 2 and 10, and one of acyl groups in the structure forms an amide bond with the α-amino group of N-terminal amino acid residue of the A-chain (the sequence shown in SEQ ID NO.1) or B-chain of the parent insulin or analog thereof or the ε-amino group of the lysine residue of the B-chain;
X is a diamino compound comprising a carboxylic acid group, which form an amide bond by connecting one of its amino groups with an acyl group in W;
Y is -D(CH₂)ₘ-, wherein m is an integer between 6 and 32, preferably an integer between 10 and 16, more preferably an integer between 12 and 14, and D is absent or CO-;
Z is -COOH;
the composition further comprises at least one component selected from the group consisting of a stabilizer, a preservative, a pH regulator and an osmotic pressure regulator.

Preferably, W forms an amide bond with the ε-amino group of the lysine residue of the B-chain. Preferably, n is an integer between 2 and 5, preferably 2.

Human insulin with threonine deletion at position 30 of the B chain is a human insulin analog, wherein the amino acid sequences of the A and B chains are as follows:

| | |
|---|---|
| A chain: GIVEQCCTSICSLYQLENYCN | SEQ ID NO. 1 |
| B chain: FVNQHLCGSHLVEALYLVCGERGFFYTPK | SEQ ID NO.2. |

The diamino compound containing a carboxylic acid group represented by X can be -HN(CH₂)pCH(COOH)NH-, wherein p is an integer between 2 and 10, preferably an integer between 2 and 6, more preferably an integer between 2 and 4, most preferably 4.

In a particularly preferred embodiment of the present invention, -W-X-Y-Z has the following structure:

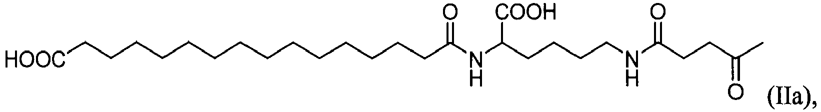

Preferably

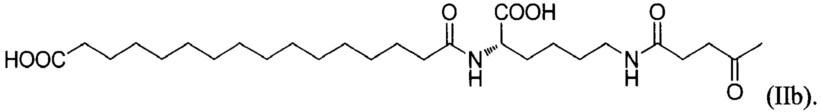

A particularly preferred acylated derivative of human insulin analog in the present invention can be customarily named as N^{α}-(HOOC(CH₂)₁₄CO)-N^{ε}-(OCCH₂CH₂CO-(N^{εB29}-Des(B30) human insulin))-Lys-OH or B29 (N^{ε}-(N^{α}-hexadecanedioic acid-L-lysine-N^{ε}-oxobutanoyl)) Des(B30) human insulin, which has a specific structure of the following formula (Ia), 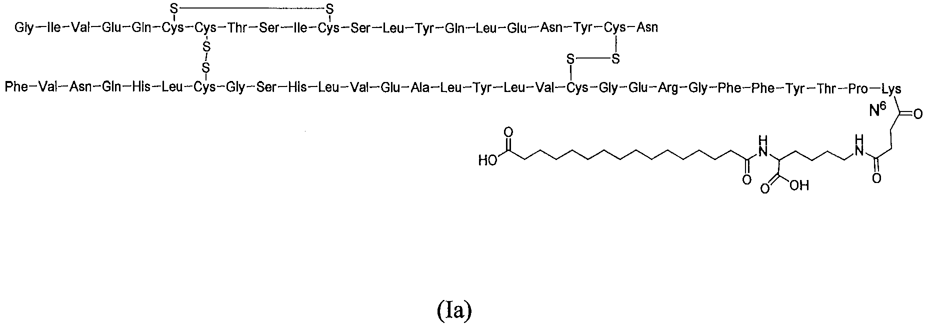 more preferably, it has a structure of (Ib).

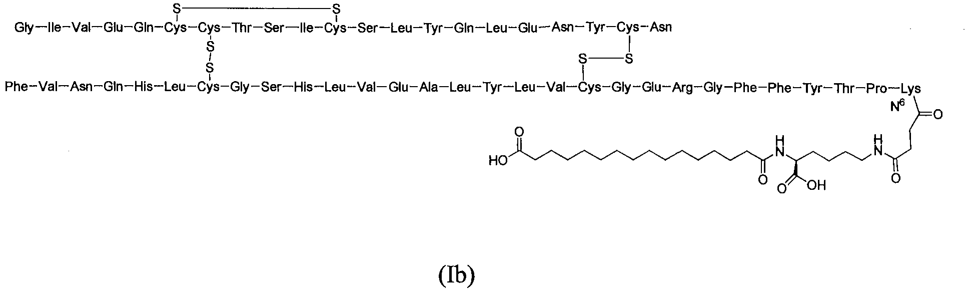

In a particularly preferred embodiment of the present invention, the acylated derivative of human insulin analog forms a complex with Zinc, which is a 6-mer of an acylated derivative of human insulin analog, wherein each 6-mer contains more than 4 zinc atoms, namely 6 molecules of acylated insulin contain more than 4 zinc atoms; more preferably 5 to 8 zinc atoms, particularly preferably 5 zinc atoms

In a preferred embodiment of the present invention, the stabilizer is selected from but not limited to sodium chloride, and the concentration of sodium chloride is preferably 5-20 mM, more preferably 5 mM, 6 mM, 7 mM, 8 mM, 9 mM, 10 mM, 11 mM, 12 mM, 13mM, 14mM, 15mM, 20mM, most preferably 10mM. When referring to the concentration of sodium chloride in the present invention, the concentration refers to the concentration of sodium chloride in the final product.

In another embodiment of the present invention, the preservative is selected from but not limited to phenol and/or m-cresol; the pH regulator is selected from acid and/or alkali, preferably but not limited to hydrochloric acid and/or sodium hydroxide; the osmotic pressure regulator is selected from but not limited to glycerol and/or mannitol.

In some embodiments of the present invention, the zinc can be any zinc salt, preferably zinc acetate.

In a preferred embodiment of the present invention, the composition comprises:
0.1%∼0.5%, preferably 0.3%∼0.4% of Lysine B29 (N^{ε}-(N^{α}-hexadecanedioic acid-L-lysine-N^{ε}-oxobutanoyl)) Des(B30) human insulin;
0.05%∼0.5%, preferably 0.1%∼0.2% of phenol;
0.05%∼0.5%, preferably 0.1%∼0.2% of m-cresol;
1%∼3%, preferably 1.5%∼2% of glycerol;
0.01%∼0.2%, preferably 0.05%∼0.06% of sodium chloride;
wherein every 6 molecules of acylated insulin contains more than 4 zinc atoms;
pH regulator and water for injection.
pH value is preferably 6-8, more preferably 7.4-7.8, most preferably 7.6.

The content of the present invention refers to the mass (g) volume (100 mL) fraction of each component in the total volume of the composition according to the total volume of the composition, for example, content of 0.05% refers to the content of 0.05g substance in a 100 mL of solution, and so on for the contents of others.

In a preferred embodiment of the present invention, the composition comprises:

| | |
|---|---|
| Lysine B29 (N^{ε}-(N^{α}-hexadecanedioic acid-L-lysine-N^{ε}-oxobutanoyl)) Des(B30) human insulin | 3.72mg; |
| phenol | 1.5mg; |
| m-cresol | 1.72mg; |
| glycerol | 19.6mg; |
| sodium chlori de | 0.58mg; |
| zinc acetate | 109.76µg; |

sodium hydroxide; hydrochloric acid; make up to 1 ml with water for injection.

pH value is preferably 6-8, more preferably 7.4-7.8, most preferably 7.6.

In an embodiment of the present invention, the composition further comprises pharmaceutically acceptable carrier.

In some embodiments of the present invention, the composition may contain fast-acting insulin in addition to the acylated derivative of the above-mentioned human insulin analog. The fast-acting insulin, also known as ultra-short-acting insulin, has a short acting time after injection. It takes effect 10 to 15 minutes after subcutaneous injection, with a peak time of 1 to 2 hours and a duration of 4 to 6 hours. Conventional fast-acting insulins in the art includes, e.g, insulin aspart (for example, NovoRapid) and insulin lispro (for example, Humalog).

The present invention also provides a method for preparing the pharmaceutical composition, which comprises a step of mixing an acylated derivative of insulin analog with any one or more selected from the group consisting of a stabilizer, a preservative, a pH regulator, an osmotic pressure regulator.

The invention also provides a use of the pharmaceutical composition in the preparation of a medicament for treating diabetes.

The specific dosage form of the pharmaceutical composition of the present invention does not need to be limited.

In addition, the present invention provides a method for treating diabetes, which comprises administering the pharmaceutical composition of the acylated derivative of human insulin analog to a patient in need of treatment.

In the present invention, the preservative and the bacteriostatic agent can have the same meaning.

### Brief description of the drawings

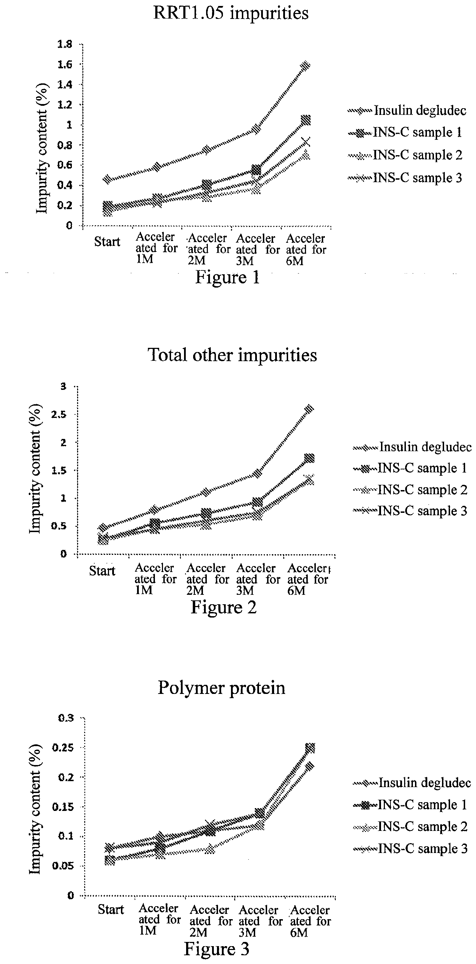

Figure 1 shows inspection result of accelerated stability of insulin degludec injection and INS-C injection at 25°C±2°C - RRT1.05 impurities.
Figure 2 shows inspection result of accelerated stability of insulin degludec injection and INS-C injection at 25°C±2°C - total other impurities.
Figure 3 shows inspection result of accelerated stability of insulin degludec injection and INS-C injection at 25°C±2°C - polymer protein.

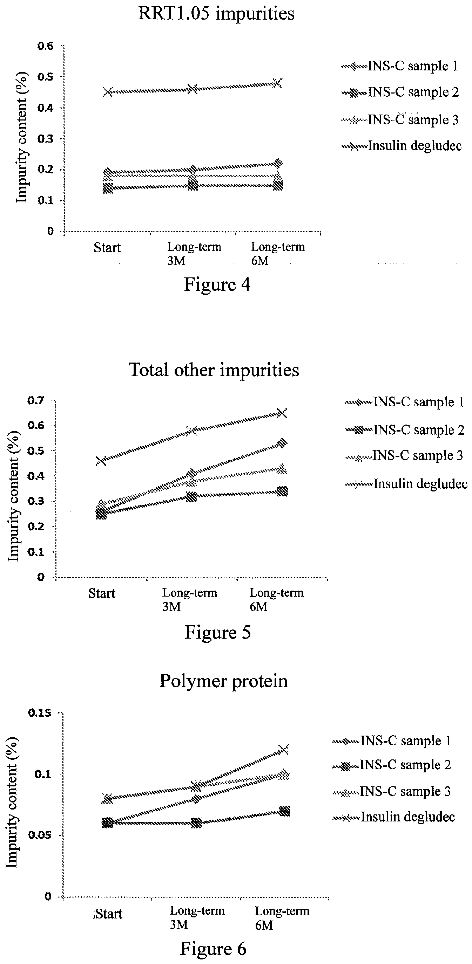

Figure 4 shows inspection result of long-term stability of insulin degludec injection and INS-C injection at 25°C±2°C - RRT1.05 impurities.
Figure 5 shows inspection result of long-term stability of insulin degludec injection and INS-C injection at 25°C±2°C - total other impurities.
Figure 6 shows inspection result of long-term stability of insulin degludec injection and INS-C injection at 25°C±2°C - RRT1.05 impurities of polymer protein.
Figure 7 shows the blood glucose level change curve of STZ rats after administration within 0-24 hours.
Figure 8 shows the plasma concentration-time curve of SD rats after administration.

### Detailed description of the preferred embodiment

The present invention is further described in detail by the following embodiments and experimental examples. These embodiments and experimental examples are only for illustrative purpose and not intended to limit the scope of the present invention. Experimental methods without specifying certain conditions in the embodiments of the present invention are generally in accordance with the conventional conditions beneficial for manufacture or the conditions proposed by the manufacturers of raw materials or commodities. Reagents are commercially available conventional reagents unless otherwise specified.

### Embodiment 1: Preparation of Lysine B29 (N^{ε}-(N^{α}-hexadecanedioic acid-L-lysine-N^{ε}-oxobutanoyl)) Des(B30) human insulin

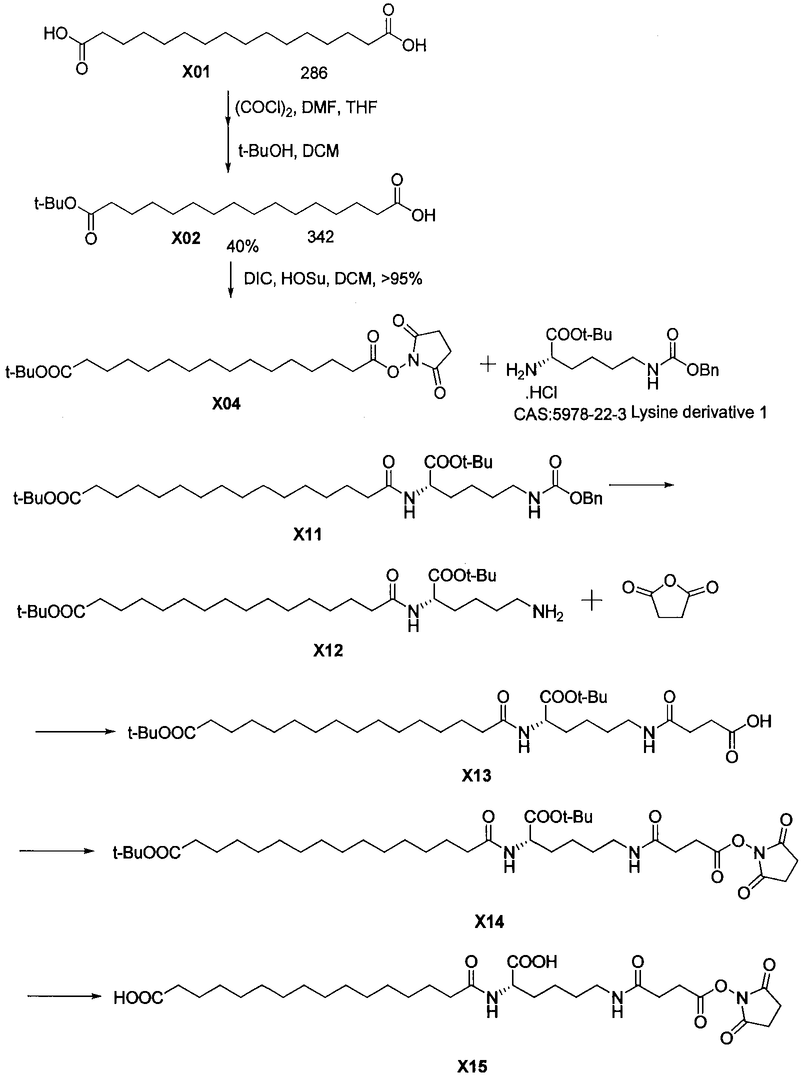

### 1. Preparation of N^{α}-(hexadecanedioic acid)-N^{ε}-(3-acylpropionic acid-OSu) lysine

X01 (150 g, 524.5 mmol) was added into dry THF (2.5 L) at room temperature, then catalytic amount of DMF (1.0 mL) was added, and oxalyl chloride (49 mL) was poured into a 100 mL constant pressure dropping funnel to slowly dropwise added into the reaction bottle. During about two hours of adding oxalyl chloride, gas was generated, which needs to be continuously deflated. After the dropwise addition was completed, the mixture was stirred at room temperature for 1.5 h, and then THF was spin-dried under reduced pressure. DCM (800 mL) and tert-butanol (500 mL) were added to the reaction flask and stirring was performed at room temperature overnight. Tert-butanol and methylene chloride were spin-dried, then methylene chloride (1 L) was added, and the mixture was filtered to remove insoluble solids. The filtrate was spin-dried and purified by column. The required components were collected, spin-dried, and recrystallized with petroleum ether to obtain 54 g of product X02, while recovering the di-tert-butyl ester product X03.

X02 (28.5 g, 83.3 mmol) was dissolved in DCM (200 mL), N-hydroxysuccinimide (5.54 g, 48.1 mmol) and diisopropylcarbodiimide (7.6 mL) were added at room temperature. After stirring at room temperature for 1 day, TLC showed that the reaction was basically completed. Then the mixture was filtered to remove insoluble solids, the solvent was spin-dried under reduced pressure, and the mixture was purified by column chromatography to obtain 28.5 g of X04.

X04 (22 g, 50.1 mmol) was dissolved in dry THF (250 mL), and stirred at room temperature. Lysine derivative (20.5 g, 55 mmol) and triethylamine (21 mL) were added to the reaction system respectively, and stirred at room temperature for 24h. The reaction system was filtered through diatomite, washed with THF three times. Then the solvent was spin-dried, and the mixture was purified by column chromatography to obtain 31 g of product X11.

X11 (30.6 g, 46.3 mmol) was dissolved in absolute ethanol (200 mL), stirred at room temperature, and 6.0 g of 10% Pd/C was added. Then hydrogen gas was filled, and the mixture was stirred vigorously and reacted at room temperature overnight. The mixture was suction filtered with diatomite and washed with absolute ethanol three times, and the filtrate was spin-dried to obtain 24.5 g of crude X12, which was directly used in the next reaction.

X12 (24 g, 45.6 mmol) was dissolved in dry THF (200 mL), triethylamine (12.7 mL) was added, follow by cooling the temperature to 0 °C. Succinic anhydride (5.2 g, 52 mmol) was added to the reaction system in batches, continuously stirred at 0 °C for 30 min, and then transferred to room temperature and stirred overnight. The THF was spin-dried under reduced pressure, and the residue was dissolved in dichloromethane (500 mL), washed twice with 5% citric acid solution (500 mL^{∗}2) and once with saturated brine, and dried over anhydrous sodium sulfate. The solvent was spin-dried under reduced pressure to obtain 29.0 g of crude X13, which was directly used in the next reaction.

X13 (28.5 g, 45.5 mmol) was dissolved in DCM (200 mL), N-hydroxysuccinimide (5.54 g, 48.2 mmol) and diisopropylcarbodiimide (7.6 mL) were added at room temperature. After stirring at room temperature for 1 day, TLC showed that the reaction was basically complete. Then the mixture was filtered to remove insoluble solids, the solvent was spin-dried under reduced pressure, and the mixture was purified by column chromatography to obtain 28.5 g of product X14.

X14 (3.0 g, 4.1 mmol) was dissolved in trifluoroacetic acid (15 mL) and stirred at room temperature for 45 min. Then trifluoroacetic acid was spin-dried under reduced pressure at low temperature, anhydrous ether was added to obtain solid precipitates that was subsequently filtered. The filter cake was washed three times with anhydrous ether and the solid was dried to obtain 1.8 g of product X15.

### 2. Preparation of Lysine B29 (N^{ε}-(N^{α}-hexadecanedioic acid-L-lysine-N^{ε}-oxobutanoyl)) Des(B30) human insulin

53 mg of human insulin (8 mg/mL, 50 mM Tris HCl pH8.5 buffer) with threonine deletion at position 30 of the B chain was taken and the pH of which was adjusted to about 10.75 with 1.5 M Na₂CO₃, and then a buffer (50 mM Tris HCl pH8.5 buffer) was used to make a constant volume of 4 mg/mL. After suspending N^{α}-(hexadecanedioic acid)-N^{ε}-(3-acylpropionic acid-OSu) lysine (18 mg) in 7 mL of acetonitrile, 1.75 mL of this solution was added to the solution of human insulin with threonine deletion at position 30 of the B chain every 15 min to start the reaction while stirring. These were done in 4 batches, and timing was started once the addition was completed. After 1 hour of reaction, the pH of the solution was adjusted to about 7.5 with acetic acid to terminate the reaction and obtain a crude solution. RP-HPLC was used to control the reaction process.

### 3. Purification of Lysine B29 (N^{ε}-(N^{α}-hexadecanedioic acid-L-lysine-N^{ε}-oxobutanoyl)) Des(B30) human insulin

The crude solution containing precursor was diluted with water to make the organic phase content about 15% (v:v), filtered with a 0.45 µm filter membrane and purified by RP-HPLC to obtain a purified solution.

### 4. Ultrafiltration and lyophilization of Lysine B29 (N^{ε}-(N^{α}-hexadecanedioic acid-L-lysine-N^{ε}-oxobutanoyl)) Des(B30) human insulin

The purified solution was replaced with water for injection using an ultrafiltration membrane package system and lyophilized to obtain 26 mg of lyophilized product. The structural formula of the obtained molecule is as follows.

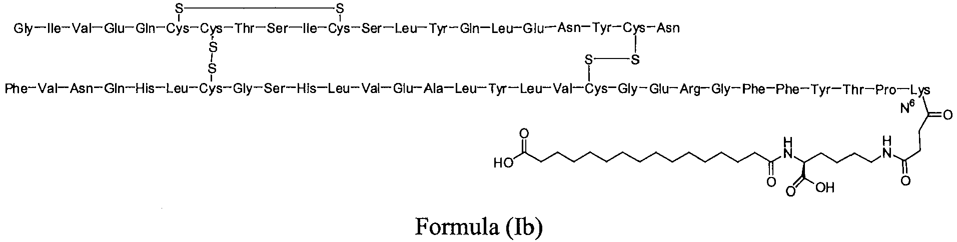

### 5. Confirmation of the structure of Lysine B29 (N^{ε}-(N^{α}-hexadecanedioic acid-L-lysine-N^{ε}-oxobutanoyl)) Des(B30) human insulin

The molecular weight measured by mass spectrum of Lysine B29 (N^{ε}-(N^{α}-hexadecanedioic acid-L-lysine-N^{ε}-oxobutanoyl)) Des(B30) human insulin was 6203.37Da, which is consistent with the theoretical molecular weight of 6203.21Da.

The target product was digested with V8 protease and LC-MS analysis of the enzymolytic product showed that a total of 4 peptide fragments were produced, with molecular weights of 416.23Da (A1-A4), 2968.29Da (A5-A17, B1-B13), 1376.57Da (A18-A21, B14-B21) and 1510.84Da (B22-B29), respectively, which are consistent with the theoretical molecular weight of peptide fragments. Among them, the B22-B29 peptide fragment is a peptide fragment modified with fatty acid chain. LC-MS/MS was used to analyze the B22-B29 peptide by secondary mass spectrometry, and the mass spectrometry confirmed that the modification site of N^{α}-hexadecanedioic acid-L-lysine-N^{ε}-oxobutanoyl was lysine at position B29 (see the table below for details). In addition, when the Edman degradation was used to sequence the B-chain amino acids, the 29th cycle could not correspond to the standard amino acid peak since the lysine at position B29 was modified. The experimental results were in line with expectations. The above experimental results jointly confirmed that the modification sites were consistent with expectations.

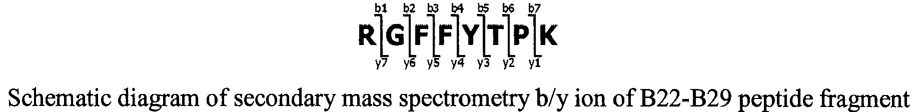

**Table of secondary mass spectrometry b/y ion of B22-B29 peptide fragment**

| b/y ion | | Charge number/theoretical value (Da) | | | | | | Measured value (Da) | Deviation (ppm) |
|---|---|---|---|---|---|---|---|---|---|
| b1/y7 | b1 | 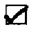 M+/ | 157.1089 | □M2+/ | 79.0581 | □M3+/ | 53.0412 | 157.1075 | -8.91 |
| | y7 | □M+/ | 1355.749 7 | □M2+/ | 678.3785 | □M3+/ | 452.5881 | - | - |
| b2/y6 | b2 | 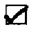 M+/ | 214.1304 | □M2+/ | 107.5688 | □M3+/ | 72.0483 | 214.1279 | -11.68 |
| | y6 | □M+/ | 1298.728 3 | □M2+/ | 649.8678 | □M3+/ | 433.5809 | - | - |
| b3/y5 | b3 | 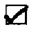 M+/ | 361.1988 | □M2+/ | 181.1030 | □M3+/ | 121.0711 | 361.1981 | -1.94 |
| | y5 | 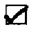 M+/ | 1151.659 8 | □M2+/ | 576.3336 | □M3+/ | 384.5581 | 1151.6501 | -8.42 |
| b4/y4 | b4 | 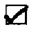 M+/ | 508.2672 | □M2+/ | 254.6373 | □M3+/ | 170.0939 | 508.2689 | 3.34 |
| | y4 | 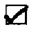 M+/ | 1004.591 4 | □M2+/ | 502.7994 | □M3+/ | 335.5353 | 1004.5888 | -2.59 |
| b5/y3 | b5 | 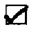 M+/ | 671.3306 | □M2+/ | 336.1689 | □M3+/ | 224.4484 | 671.3277 | -4.32 |
| | y3 | 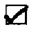 M+/ | 841.5281 | □M2+/ | 421.2677 | □M3+/ | 281.1809 | 841.5231 | -5.94 |
| b6/y2 | b6 | 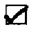 M+/ | 772.3782 | □M2+/ | 386.6928 | □M3+/ | 258.1309 | 772.3756 | -3.37 |
| | y2 | 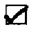 M+/ | 740.4804 | □M2+/ | 370.7438 | □M3+/ | 247.4983 | 740.4792 | -1.62 |
| b7/y1 | b7 | 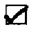 M+/ | 869.4310 | □M2+/ | 435.2191 | □M3+/ | 290.4819 | 869.4268 | -4.83 |
| | y1 | 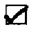 M+/ | 643.4277 | □M2+/ | 322.2175 | □M3+/ | 215.1474 | 643.4223 | -8.39 |

### Embodiment 2: Preparation of INS-C injection

INS-C represents Lysine B29 (N^{ε}-(N^{α}-hexadecanedioic acid-L-lysine-N^{ε}-oxobutanoyl)) Des(B30) human insulin in the embodiment.

The prescription of INS-C injection is as follows:

| Prescription composition | Unit prescription | 5000 bottles of prescription | Function |
|---|---|---|---|
| INS-C | 3.72 mg | 20.46 g | basic remedy |
| zinc acetate | 109.76 µg | 0.60 g | stabilizer |
| phenol | 1.5 mg | 8.25 g | bacteriostatic agent |
| m-cresol | 1.72 mg | 9.46 g | bacteriostatic agent |
| glycerol^{①} | 19.6 mg | 107.80 g^{②} | osmotic pressure regulator |
| sodium chloride | 0.58 mg | 3.19 g | stabilizer |
| sodium hydroxide | q.s. | q.s. | pH regulator |
| hydrochloric acid | q.s. | q.s. | pH regulator |
| water for injection to | 1 mL | 5500 mL | solvent |

| | | | |
|---|---|---|---|
| Note: Molecular formula of zinc acetate: C₄H₆O₄Zn•2H₂O | | | |

Solutions were respectively prepared for use according to the content of excipients in the prescription.

The excipients were mixed with the prescribed amount of INS-C active pharmaceutical ingredient, the water for injection was supplemented to 90% of the target volume, the pH was adjusted to 7.4∼7.8 with NaOH solution or HCl solution, the target pH was 7.6, then water for injection was added to the full amount, and the mixture were mixed well.

Samples were taken to examine the properties, pH, INS-C content, phenol content and m-cresol content. The standards of each index are as follows: properties: colorless clear liquid; pH: 7.4∼7.8; content of INS-C: 97.0%∼103.0%; content of phenol: 1.46∼1.55 mg/mL; content of m-cresol: 1.67∼1.77 mg/mL.

The mixture was filtered with 0.22 µm PVDF membrane, and the filtrate is filled in a 2 mL neutral borosilicate glass injection bottle according to a volume of 1.1 mL per bottle, with the filling volume of 1.05 mL to 1.15 mL. Bottles were stoppered and capped. The filling volume is monitored during the filling process, and the integrity of the filter element was tested before and after filtration. Bottles were subjected to light inspection and then packaged.

### Embodiment 3: Screening of sodium chloride concentration in INS-C injection

INS-C represents Lysine B29 (N^{ε}-(N^{α}-hexadecanedioic acid-L-lysine-N^{ε}-oxobutanoyl)) Des(B30) human insulin in the embodiment.

Prescription information is as follows:

| Prescription composition | Concentration and pH of prescription | | |
|---|---|---|---|
| | Prescription 1 | Prescription 2 | Prescription 3 |
| pH of prescription | 7.2 | 7.2 | 7.2 |
| INS-C (nmol/mL) | 600 | 600 | 600 |
| zinc acetate (nmol/mL) | 500 | 500 | 500 |
| glycerol (mg/mL) | 19.6 | 19.6 | 19.6 |
| phenol (mg/mL) | 1.5 | 1.5 | 1.5 |
| m-cresol (mg/mL) | 1.72 | 1.72 | 1.72 |
| sodium chloride (mg/mL) | 0 | 10 | 20 |

The effect of sodium chloride on the stability of compositions was evaluated and the results were as follows.

**Table 1 Effect of sodium chloride on the stability of compositions**

| Time | No. of prescription | Properties | Purity of main peak (%) | Related substance(%) | | Polymer impurities (%) |
|---|---|---|---|---|---|---|
| | | | | Total impurities between the main peak and PRT1.10 | Total other impurities except for those between the main peak and PRT1.10 | |
| Start | NaCl-0 mg/mL | Colorless, clear and transparent solution | 99.06 | 0.52 | 0.42 | 0.09 |
| | NaCl-10 mg/mL | Colorless, clear and transparent solution | 98.99 | 0.73 | 0.28 | 0.05 |
| | NaCl-20 mg/mL | Colorless, clear and transparent solution | 99 | 0.71 | 0.29 | 0.04 |
| 7d | NaCl-0 mg/mL | Colorless, clear and transparent solution | 98.15 | 1.31 | 0.54 | 0.09 |
| | NaCl-10 mg/mL | Colorless, clear and transparent solution | 98.67 | 0.97 | 0.36 | 0.05 |
| | NaCl-20 mg/mL | Colorless, clear and transparent solution | 98.71 | 1.06 | 0.23 | 0.05 |
| 14d | NaCl-0 mg/mL | Colorless, clear and transparent solution | 96.92 | 2.46 | 0.62 | 0.13 |
| | NaCl-10 mg/mL | Colorless, clear and transparent solution | 98.01 | 1.45 | 0.53 | 0.07 |
| | NaCl-20 mg/mL | Colorless, clear and transparent solution | 98.22 | 1.51 | 0.27 | 0.08 |

Table 1 shows that sodium chloride can reduce the formation of high polymers as well as the formation of other impurities, thus improving the stability of active substances.

### Embodiment 4: Stability comparison between INS-C injection and insulin degludec injection

INS-C represents Lysine B29 (N^{ε}-(N^{α}-hexadecanedioic acid-L-lysine-N^{ε}-oxobutanoyl)) Des(B30) human insulin in the embodiment.

The prescription information of INS-C injection and insulin degludec injection are as follows:

| Product | | Tresiba® | N/A | Function |
|---|---|---|---|---|
| Common name/code name | | insulin degludec injection | INS-C injection | |
| Specification | | 300 U /3mL (100 U/mL) | 1m1:3.72mg (600nmol/ml) | |
| active ingredient | | insulin degludec | INS-C | basic remedy |
| excipi ents | glycerol | 19.6mg/mL | 19.6mg/mL | osmotic pressure regulator |
| | phenol | 1.50mg/mL | 1.50mg/mL | bacteriostatic agent |
| | m-cresol | 1.72mg/mL | 1.72mg/mL | bacteriostatic agent |
| | Zinc | 32.7µg/mL | 32.7µg/mL | stabilizer |
| | sodium chloride | N/A | 0.58 mg/mL | stabilizer |
| | hydrochlori c acid | q.s. | q.s. | pH regulator |
| | sodium hydroxide | q.s. | q.s. | pH regulator |
| solvent | water for injection | added to 3ml | added to 1ml | solvent |

### A. Accelerated test

Insulin degludec injection and 3 batches of pilot test samples of INS-C injection were placed under accelerated test conditions at 25°C±2°C, and samples were taken at 0, 1, 2, 3 and 6 months respectively for determination and each index was examined.

### B. Long-term test

Insulin degludec injection and 3 batches of pilot test samples of INS-C injection were placed for long-term storage at a low temperature of 5°C±3°C, and samples were taken at 3, 6, 9, 12, 18, 24 and 36 months respectively for measurement, sampling and measuring at regular intervals. The starting point for the investigation of insulin degludec injection was calculated based on its delivery time.

**Table 2 Initial test results of insulin degludec injection and INS-C injection**

| Sample type | | | insulin degludec injection | 3 batches of pilot test samples of INS-C injection | | |
|---|---|---|---|---|---|---|
| Inspection item | | | | | | |
| pH value | | | 7.6 | 7.4 | 7.5 | 7.4 |
| Related substan ces | RRT1.05 impurities% | | 0.45 | 0.19 | 0.14 | 0.18 |
| | Total other impurities% | | 0.46 | 0.26 | 0.25 | 0.29 |
| High polymer protein (%) | | | 0.08 | 0.06 | 0.06 | 0.08 |
| Zinc (µg/mL) | | | 33.70 | 31.95 | 32.00 | 32.58 |
| phenol and m-cresol (mg/mL) | | phenol | 1.55 | 1.52 | 1.48 | 1.52 |
| | | m-cresol | 1.75 | 1.74 | 1.68 | 1.73 |
| Osmolarity (mOsmol/kg) | | | 261 | 283 | 280 | 282 |

| | | | | | | |
|---|---|---|---|---|---|---|
| Notes: The appearance of which is colorless, clear and transparent solution. | | | | | | |

**Table 3 Investigation results of accelerated stability of insulin degludec injection and INS-C injection at 25°C±2°C**

| Groups | Storage condition | pH | Phenol content (mg/ml) | M-cresol content (mg/ml) | Related substances | | High polymer protein (%) |
|---|---|---|---|---|---|---|---|
| | | | | | RRT1.05 impurities % | other impurities % | |
| insulin degludec | Start | 7.6 | 1.55 | 1.75 | 0.45 | 0.46 | 0.08 |
| | Accelerated for 1M | 7.5 | 1.53 | 1.73 | 0.58 | 0.79 | 0.10 |
| | Accelerated for 2M | 7.6 | 1.53 | 1.71 | 0.75 | 1.12 | 0.11 |
| | Accelerated for 3M | 7.5 | 1.52 | 1.71 | 0.96 | 1.45 | 0.12 |
| | Accelerated for 6M | 7.4 | 1.50 | 1.67 | 1.59 | 2.60 | 0.22 |
| INS-C sample 1 | Start | 7.4 | 1.52 | 1.74 | 0.19 | 0.26 | 0.06 |
| | Accelerated for 1M | 7.3 | 1.51 | 1.72 | 0.27 | 0.56 | 0.08 |
| | Accelerated for 2M | 7.2 | 1.50 | 1.71 | 0.41 | 0.74 | 0.11 |
| | Accelerated for 3M | 7.2 | 1.49 | 1.71 | 0.56 | 0.94 | 0.14 |
| | Accelerated for 6M | 7.2 | 1.50 | 1.71 | 1.05 | 1.72 | 0.25 |
| INS-C sample 2 | Start | 7.5 | 1.48 | 1.68 | 0.14 | 0.25 | 0.06 |
| | Accelerated for 1M | 7.4 | 1.44 | 1.59 | 0.25 | 0.45 | 0.07 |
| | Accelerated for 2M | 7.4 | 1.48 | 1.67 | 0.29 | 0.54 | 0.08 |
| | Accelerated for 3M | 7.4 | 1.46 | 1.66 | 0.37 | 0.70 | 0.12 |
| | Accelerated for 6M | 7.3 | 1.47 | 1.65 | 0.71 | 1.33 | 0.25 |
| INS-C sample 3 | Start | 7.4 | 1.52 | 1.73 | 0.18 | 0.29 | 0.08 |
| | Accelerated for 1M | 7.3 | 1.52 | 1.72 | 0.23 | 0.45 | 0.09 |
| | Accelerated for 2M | 7.3 | 1.52 | 1.71 | 0.33 | 0.61 | 0.12 |
| | Accelerated for 3M | 7.3 | 1.50 | 1.70 | 0.45 | 0.76 | 0.14 |
| | Accelerated for 6M | 7.3 | 1.50 | 1.68 | 0.83 | 1.35 | 0.25 |

| | | | | | | | |
|---|---|---|---|---|---|---|---|
| Notes: There is no significant difference between the test results of the stability of upright sample and inverted sample of the INS-C injection. Only the results of the inverted condition (relatively violent) are listed here. Wherein the appearance of which is colorless, clear and transparent solution. | | | | | | | |

**Table 4 Long-term stability of insulin degludec injection and INS-C injection (5°C±3°C)**

| Groups | Storage condition | pH | Phenol content (mg/ml) | M-cresol content (mg/ml) | Related substances | | High polymer protein (%) |
|---|---|---|---|---|---|---|---|
| | | | | | RRT1.05 impurities % | other impurities % | |
| insulin degludec | Long-term 13M | 7.6 | 1.55 | 1.75 | 0.45 | 0.46 | 0.08 |
| | Long-term 16M | 7.5 | 1.52 | 1.72 | 0.46 | 0.58 | 0.09 |
| | Long-term 19M | 7.4 | 1.52 | 1.71 | 0.48 | 0.65 | 0.12 |
| INS-C sample 1 | Start | 7.4 | 1.52 | 1.74 | 0.19 | 0.26 | 0.06 |
| | Long-term 3M | 7.2 | 1.49 | 1.72 | 0.20 | 0.41 | 0.08 |
| | Long-term 6M | 7.2 | 1.51 | 1.72 | 0.22 | 0.53 | 0.10 |
| INS-C sample 2 | Start | 7.5 | 1.48 | 1.68 | 0.14 | 0.25 | 0.06 |
| | Long-term 3M | 7.4 | 1.46 | 1.67 | 0.15 | 0.32 | 0.06 |
| | Long-term 6M | 7.4 | 1.48 | 1.67 | 0.15 | 0.34 | 0.07 |
| INS-C sample 3 | Start | 7.4 | 1.52 | 1.73 | 0.18 | 0.29 | 0.08 |
| | Long-term 3M | 7.4 | 1.50 | 1.71 | 0.18 | 0.38 | 0.09 |
| | Long-term 6M | 7.4 | 1.51 | 1.71 | 0.18 | 0.43 | 0.10 |

| | | | | | | | |
|---|---|---|---|---|---|---|---|
| Notes: There is no significant difference between the test results of the stability of upright sample and the inverted sample of the INS-C injection. Only the results of the inverted (relatively violent) condition are listed here. Wherein the appearance of which is colorless, clear and transparent solution. | | | | | | | |

### Embodiment 5: Investigation of the hypoglycemic effect of INS-C injection and insulin degludec injection on STZ-induced type 1 diabetes model

INS-C represents Lysine B29 (N^{ε}-(N^{α}-hexadecanedioic acid-L-lysine-N^{ε}-oxobutanoyl)) Des(B30) human insulin in the embodiment.

### 1. Test samples

| Name | INS-C injection | Insulin degludec injection |
|---|---|---|
| Suppliers | The present invention | Novo Nordisk |
| Physical state | Colorless liquid | Colorless liquid |
| Storage condition | 4°C | 4°C |

### 2. Preparation of test samples

The test samples were stored in the dark at 4°C under a dosage of 7.5 nmol/kg. The temperature of the drugs was restored to room temperature when administered.

### 3. Experimental animals

| Species | SD rat |
|---|---|
| Grade | SPF animal |
| Week age at purchase | 6 weeks |
| Week age at the start of the experiment | 8-9 weeks |
| Weight range | 300-400g |
| Gender | Male |
| Supplier | Beijing Vital River Laboratory Animal Technologies Co. Ltd |
| Supplier address | Beijing, China |
| Animal identification method | Mark at the tail with a marker |
| Quantity of animals ordered | 50 |
| Quantity of animals used | 29 |

### 4. Experimental method

SPF rats were raised in the laboratory environment for 7 days with standard feed and standard cages at a temperature of 20-25 °C and a humidity of 40-60%. The day before modeling, the rats were fasted for 16 hours, injected STZ (65 mg/kg) intraperitoneally and resumed feeding one hour later. After modeling, sufficient drinking water (2-3 times the normal amount of drinking water was provided) and food were given daily, and litter was changed 1-2 times daily to kept dry. Fasting blood glucose was measured on the fifth day (fasting for 6 hours), and rats with a blood glucose value> 16.7 mmol/L were selected. These rats were randomly divided into 5 groups based on blood sugar levels. Animal grouping and administration information are as follows:

| Groups | Animal type | Quantity of animals | Administration dosage (nmol /kg) | Administr ation volume (mL/kg) | Administration route | Administration frequency |
|---|---|---|---|---|---|---|
| Control | STZ rat | 10 | / | 1 | SC | once |
| insulin degludec injection | STZ rat | 10 | 7.5 | 1 | SC | once |
| INS-C injection | STZ rat | 9 | 7.5 | 1 | SC | once |

No fasting was required before administration, and each drug was injected subcutaneously. The control group was injected with the same volume of solution with INS-C removed but containing other ingredients of the INS-C prescription and performed whole course of fasting after administration. Blood glucose levels of rats were measured 1, 2, 4, 6, 8, 10, 12 and 24 hours after administration.

All data were recorded in an Excel spreadsheet by means of Mean ± SEM. SPSS software was used for statistical analysis of the data based on one way or two way ANOVA comparison method, with P <0.05 as the criterion of significant difference.

### 5. Results

The basic blood glucose level of the control group was 26.6 mmol/L before administration, and gradually decreased after administration, reaching 10.1 mmol/L after 24 hours, with a blood glucose change value of 15.5 mmol/L. The blood glucose change value of the insulin degludec positive drug group before and after administration was 20.1 mmol/L. The blood glucose levels of the insulin degludec positive drug group were significantly different from that of the control group at 1, 2, 4, 6, 8, 10 and 12 hours after administration, indicating the effectiveness of the animal model and experimental method.

Compared with the control group, the test drug INS-C could significantly reduce the animals' blood glucose level within 24 hours after administration. From the perspective of blood glucose value, the blood glucose value of the test drug group 3.9±0.6 mmol/L) was lower than that of the control group (10.1±3.3 mmol/L) and also lower than that of insulin degludec (6.4±1.4 mmol/L), indicating that INS-C injection had a good long-term hypoglycemic effect. The differences in blood glucose reduction within 0 to 24 hours of each group were compared, and the specific values are shown in Table 5:

**Table 5 Effect of single administration on blood glucose of STZ-induced type I diabetic rats**

| Groups | Dosage ( nmol/kg) | Blood glucose level (mmol/L) | | | | | | | | |
|---|---|---|---|---|---|---|---|---|---|---|
| | | 0 h | 1 h | 2 h | 4 h | 6 h | 8 h | 10 h | 12 h | 24 h |
| Control | - | 26.6±0.9 | 23.7±0.8 | 21.5±0.7 | 21.9±1.5 | 20.2±2.8 | 15.4±2.7 | 13.3±2.9 | 15.0+3.2 | 10.1±3.3 |
| INS-C | 7.5 | 26.4±0.9 | 21.9±0.7 | 17.9±1.4 | 4.8±1.3** | 2.2±0.1** | 2.3±0.2** | 2.6±0.3** | 2.9±0.5** | 8.9±0.6* |
| insulin degludec | 7.5 | 26.5±0.9 | 20.8±0.8 | 15.2±1.9* | 3.2±0.6** | 2.2±0.3** | 2.5±0.4** | 3.8±0.5** | 4.2±0.5** | 6.4±1.4 |

| | | | | | | | | | | |
|---|---|---|---|---|---|---|---|---|---|---|
| Note: Control group, insulin degludec group: N=10, INS-C group: N=9; **P<0.01 vs control group | | | | | | | | | | |

According to the same experimental method, the effect of INS-C injection without sodium chloride and insulin degludec injection on the hypoglycemic effect of rats was compared, and the results showed that the hypoglycemic curve of which was basically the same as that of above experiment, proving that the presence or absence of sodium chloride had little effect on the exposure of INS-C.

### Example 6: Investigation of the pharmacokinetic properties of sodium chloride-containing preparation, sodium chloride-free preparations and sodium chloride-containing preparation after being kept at room temperature for 20 hours in normal rats

**Laboratory animal**

| Species | SD rat |
|---|---|
| Grade | SPF animal |
| Week age at purchase | 6 weeks |
| Week age at the start of the experiment | 8-9 weeks |
| Weight range | 300-400g |
| Gender | Male |
| Supplier | Beijing Vital River Laboratory Animal Technologies Co. Ltd |
| Supplier address | Beijing, China |
| Animal identification method | Mark at the tail with a marker |
| Quantity of animals ordered | 15 |
| Quantity of animals used | 15 |

1. Experimental method: SD rats were acclimatized to animal room for 5 days and were fed with standard feed and standard cages at a temperature of 20-25 °C and a humidity of 40-60%. Before administration, they were randomly divided into three groups according to body weight. The grouping information is as follows:

| Group No. | Group | Quantity of animals | Administration dosage (nmol/kg) | Administration volume (mL/kg) | Administration route | Administration frequency |
|---|---|---|---|---|---|---|
| 1 | sodium chloride-free preparations | 5 | 50 | 1 | SC | once |
| 2 | sodium chloride-containing preparations | 5 | 50 | 1 | SC | once |
| 3 | sodium chloride-containing preparations after being kept at room temperature for 20 hours | 5 | 50 | 1 | SC | once |

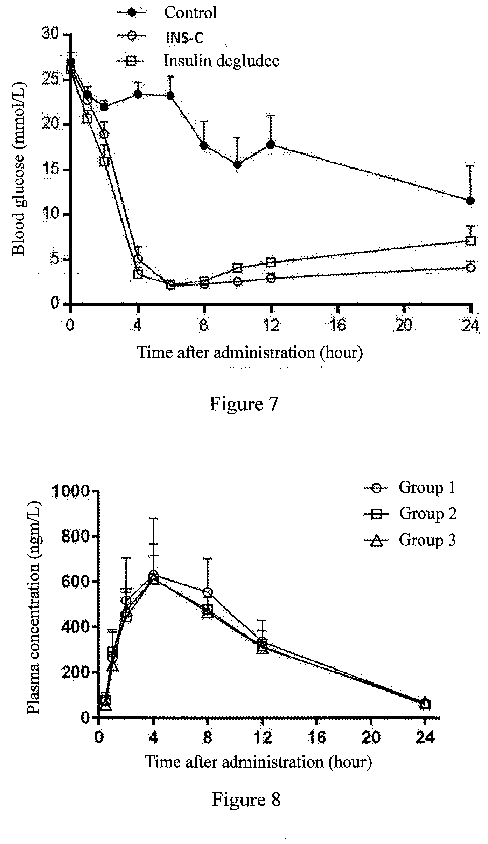

Group 1 and Group 2: the sodium chloride-free INS-C preparation (600 nmol/mL) or sodium chloride-containing INS-C preparation (600 nmol/mL) was diluted to 50 nmol/mL with blank solvent before administration; Group 3: sodium chloride-containing INS-C preparation (600 nmol/mL) was kept in the dark and room temperature environment for 20 hours, then diluted to 50 nmol/mL with a blank solvent before administration, wherein the sodium chloride concentration in the sodium chloride group was 0.58 mg/mL. No fasting is required before administration, and each test drug was injected subcutaneously once. Blood sample was collected from each animal via jugular vein puncture at about 0.2 mL/time point before and 0.5, 1, 2, 4, 8, 12 and 24 hours after administration, and placed in a test tube containing coagulant. Blood sample was collected and storage in a labeled centrifuge tubes, and serum was centrifuged and separated (centrifugation conditions: 5000 rpm, 10 minutes, 2∼8°C).
Serum samples were measured for drug concentration by LC-MS/MS, the main pharmacokinetic parameters were calculated, and student t test was used to analyze statistical differences.
2. Experiment results: The average blood drug concentration at each timing (see Table 7 for individual blood drug concentration) is plotted as the ordinate and the time point is plotted as the abscissa to obtain the blood drug concentration-time curve (Figure 8). The results show that the trend of the concentration of the test drugs in the three groups of animals over time is basically the same. The main pharmacokinetic parameters are shown in Table 6. The results show that the main pharmacokinetic parameters among the groups are basically the same, with no statistical difference.

The experiment results show that the addition of sodium chloride to the preparation has no significant effect on the pharmacokinetic properties of rats; being kept at room temperature for 20 hours has no significant effect on the pharmacokinetic properties of the test drugs.

**Table 6 Main pharmacokinetic parameters of SD rats after administration (Mean±SD)**

| **Group** | **tₘₐₓ (h)** | **Cₘₐₓ (ng/mL)** | **AUC₀₋ₜ (ng*h/mL)** | **t_{1/2} (h)** | **MRT (h)** |
|---|---|---|---|---|---|
| 1 | 5.6±2.2 | 712.8±181.4 | 8209±1149 | 5.3±0.6 | 10.2±0.9 |
| 2 | 4.8±1.8 | 622.3±146.6 | 7585±478 | 5.3±0.7 | 10.2±1.6 |
| 3 | 4.0±0.0 | 614.5±102.2 | 7767±474 | 5.8±0.8 | 10.8±1.4 |

**Table 7 Individual blood drug concentration (ng/mL) of SD rats after administration**

| **Group** | **Animal No.** | **Timing** | | | | | | | |
|---|---|---|---|---|---|---|---|---|---|
| | | **0 h** | **0.5 h** | **1 h** | **2 h** | **4 h** | **8 h** | **12 h** | **24 h** |
| **1** | **101** | 0 | 36.483 | 113.263 | 286.035 | 342.130 | 725.746 | 476.846 | 69.587 |
| | **102** | 0 | 59.099 | 188.470 | 535.247 | 565.447 | 476.356 | 264.303 | 50.088 |
| | **103** | 0 | 98.046 | 377.288 | 694.382 | 901.454 | 387.275 | 237.355 | 63.335 |
| | **104** | 0 | 76.265 | 277.108 | 365.509 | 472.513 | 495.033 | 367.180 | 68.893 |
| | **105** | 0 | 98.269 | 365.144 | 706.402 | 876.076 | 694.095 | 342.620 | 74.279 |
| **2** | **201** | 0 | 106.498 | 350.589 | 425.646 | 742.027 | 522.003 | 293.174 | 53.690 |
| | **202** | 0 | 119.645 | 414.173 | 573.117 | 807.472 | 462.107 | 219.074 | 34.882 |
| | **203** | 0 | 55.535 | 228.971 | 311.823 | 463.187 | 393.794 | 368.933 | 66.588 |
| | **204** | 0 | 80.621 | 300.338 | 533.602 | 575.937 | 489.009 | 303.847 | 58.295 |
| | **205** | 0 | 39.949 | 167.879 | 388.326 | 482.801 | 522.915 | 397.095 | 86.314 |
| **3** | **301** | 0 | 82.628 | 281.699 | 534.397 | 699.607 | 503.430 | 315.331 | 57.336 |
| | **302** | 0 | 28.450 | 145.201 | 352.891 | 482.749 | 454.196 | 362.401 | 101.844 |
| | **303** | 0 | 53.603 | 232.594 | 428.889 | 550.151 | 498.077 | 271.003 | 59.075 |
| | **304** | 0 | 78.973 | 284.140 | 595.609 | 728.647 | 451.208 | 315.893 | 65.198 |
| | **305** | 0 | 53.117 | 222.510 | 466.115 | 611.153 | 433.111 | 290.685 | 68.910 |

Although the specific embodiments of the present invention have been described above, It is to be understood by those skilled in the art that the foregoing embodiments are merely illustrative, and various changes or modification can be made to these embodiments without departing from the principle and essence of the present invention. Therefore, the protection scope of the present invention is defined by the appended claims.

## Claims

1. A pharmaceutical composition comprising an acylated derivative of human insulin analog, wherein the acylated derivative of human insulin analog has a structure represented by the following general formula I:
S-W-X-Y-Z (I)
wherein S is a human insulin with threonine deletion at position 30 of the B chain; -W-X-Y-Z is an acylation modification group of the insulin analog;
wherein W is a diacyl structure with -OC(CH₂)ₙCO-, where n is an integer between 2 and 10, and one of acyl groups in the structure forms an amide bond with the α-amino group of N-terminal amino acid residue of the A-chain (the sequence shown in SEQ ID NO.1) or B-chain of the parent insulin or analog thereof or the ε-amino group of the lysine residue of the B-chain;
X is a diamino compound comprising a carboxylic acid group, which form an amide bond by connecting one of its amino groups with an acyl group in W;
Y is -D(CH₂)ₘ-, wherein m is an integer between 6 and 32, preferably an integer between 10 and 16, more preferably an integer between 12 and 14, and D is absent or CO-;
Z is -COOH;
the composition also comprises at least one component selected from the group consisting of stabilizer, preservative, pH regulator and osmotic pressure regulator.

2. The pharmaceutical composition of claim 1, wherein W of the acylated derivative of the human insulin analog forms an amide bond with the ε-amino group of the lysine residue of the B-chain.

3. The pharmaceutical composition of claim 1 or 2, wherein n of the acylated derivative of human insulin analog is an integer between 2 and 5, preferably 2.

4. The pharmaceutical composition of any one of claims 1 to 3, wherein X of the acylated derivative of human insulin analog is -HN(CH₂)ₚCH(COOH)NH-, and p is an integer between 2 and 10, preferably an integer between 2 and 6, more preferably an integer between 2 and 4, most preferably 4.

5. The pharmaceutical composition of any one of claims 1 to 4, wherein -W-X-Y-Z of the acylated derivative of human insulin analog has the following structure: 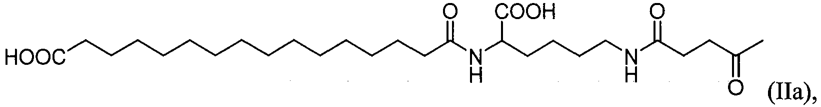 preferably

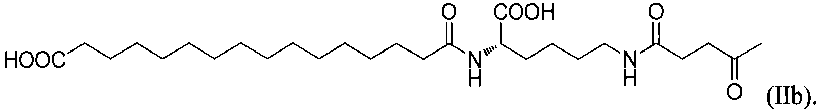

6. The pharmaceutical composition of any one of claims 1 to 5, wherein the acylated derivative of human insulin analog forms a complex with Zinc; preferably, wherein every 6 molecules of acylated insulin contains more than 4 zinc atoms.

7. The pharmaceutical composition of any one of claims 1 to 6, wherein the stabilizer is selected from sodium chloride, and the concentration of the sodium chloride is preferably 5-20 mM, more preferably 10 mM.

8. The pharmaceutical composition of any one of claims 1 to 7, wherein the preservative is selected from phenol and/or m-cresol; the pH regulator is selected from acid and/or alkali; the osmotic pressure regulator is selected from glycerin and/or mannitol.

9. The pharmaceutical composition of claim 8 comprising the following components:
0.1%∼0.5%, preferably 0.3%∼0.4% of Lysine B29 (N^{ε}-(N^{α}-hexadecanedioic acid-L-lysine-N^{ε}-oxobutanoyl)) Des(B30) human insulin;
0.05%∼0.5%, preferably 0.1%∼0.2% of phenol;
0.05%∼0.5%, preferably 0.1%∼0.2% of m-cresol;
1%∼3%, preferably 1.5%∼2% of glycerin;
0.01%∼0.2%, preferably 0.05%∼0.06% of sodium chloride;
wherein every 6 said insulin contains more than 4 zinc atoms;
the pH regulator and water for injection, preferably have a pH value of 6-8, more preferably 7.4-7.8.

10. The pharmaceutical composition of claim 8 comprising the following components:
| | |
|---|---|
| Lysine B29 (N^{ε}-(N^{α}-hexadecanedioic acid-L-lysine-N^{ε}-oxobutanoyl)) Des(B30) human insulin | 3.72mg; |
| Phenol | 1.5mg; |
| M-cresol | 1.72mg; |
| Glycerin | 19.6mg; |
| Sodium chloride | 0.58mg; |
| Zinc acetate | 109.76µg; |
Sodium hydroxide; hydrochloric acid; make up to 1 ml with water for injection, preferably has a pH value of 6-8, more preferably 7.4-7.8, most preferably 7.6.

11. The pharmaceutical composition of any one of claims 1 to 10, further comprising fast-acting insulin.

12. A method for preparing the pharmaceutical composition of any one of claims 1 to 10, which comprises a step of mixing an acylated derivative of insulin analog with any one or more selected from the group consisting of a stabilizer, a preservative, a pH regulator, and an osmotic pressure regulator.

13. A use of the pharmaceutical composition of any one of claims 1 to 11 in the preparation of a medicament for treating diabetes.

14. A method for treating diabetes, which comprises administering the pharmaceutical composition of any one of claims 1 to 11 to a patient in need of treatment.
